(19) Europäisches Patentamt
European Patent Office
Office européen des brevets

(11) Veröffentlichungsnummer: **0 257 615 B1**

(12) **EUROPÄISCHE PATENTSCHRIFT**

(45) Veröffentlichungstag der Patentschrift: **04.12.91**

(51) Int. Cl.$^5$: **C07H 17/08**, A61K 31/70, C12P 19/62, //(C12P19/62, C12R1:465)

(21) Anmeldenummer: **87112298.2**

(22) Anmeldetag: **25.08.87**

# (54) Ein neues Makrolid-Antibiotikum, ein mikrobiologisches Verfahren zu seiner Herstellung und seine Verwendung als Arzneimittel.

(30) Priorität: **27.08.86 DE 3629063**

(43) Veröffentlichungstag der Anmeldung:
**02.03.88 Patentblatt 88/09**

(45) Bekanntmachung des Hinweises auf die Patenterteilung:
**04.12.91 Patentblatt 91/49**

(84) Benannte Vertragsstaaten:
**AT BE CH DE ES FR GB GR IT LI LU NL SE**

(56) Entgegenhaltungen:

**THE JOURNAL OF ANTIBIOTICS, Band 29, Nr. 4, April 1976, Seite 76-22, Japan Antibiotics Research Association, Tokyo, JP; "Aldagamycin F"**

(73) Patentinhaber: **HOECHST AKTIENGESELLSCHAFT**
**Postfach 80 03 20**
**W-6230 Frankfurt am Main 80(DE)**

(72) Erfinder: **Franco, Christopher Milton Mathew, Dr.**
**74A, The Westminister N.S. Mankikar Marg**
**Sion Bombay 400 022(IN)**
Erfinder: **Gandhi, Julia Charishma**
**128K Hill Road Somnath Lane**
**Bandra Bombay 400 050(IN)**
Erfinder: **Chatterjee, Sugata**
**H1/2 Hoechst Quarters Darga Road**
**Mulund (West) Bombay 400 082(IN)**
Erfinder: **Reddy, Goukanapalli Chandra Shekara**
**104 Aarti Apartments Sarojini Naidu Road**
**Mulund (West) Bombay 400 080(IN)**

EP 0 257 615 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

THE JOURNAL OF ANTIBIOTICS, Band 38, Nr. 4, April 1985, Seiten 522-523, Japan Antibiotics Research Association, Tokyo, JP; K. KINOSHITA et al.: "Mycinamicins, new macrolide antibiotics VIII. Chemical degradation and absolute configuration of mycinamincins"

THE JOURNAL OF ANTIBIOTICS, Band 51, Nr. 10, Oktober 1987, Seiten 1368-1374, Japan Antibiotics Research Association, Tokyo, JP; S. CHATTERJEE et al.: "Swalpamycin, a new macrolide antibiotic II. Structure elucidation"

Erfinder: Ganguli, Bimal Naresh, Dr.
Saurayuth 173 Central Avenue
Chembur Bombay 400 071(IN)
Erfinder: Rupp, Richard Helmut, Dr.
Niladri 66 Nepean Sea Road
Bombay 400 006(IN)
Erfinder: Kogler, Herbert
Breslauer Strasse 39
W-6233 Kelkheim (Taunus)(DE)
Erfinder: Fehlhaber, Hans-Wolfram, Dr.
Thomas-Mann-Strasse 5a
W-6270 Idstein/Taunus(DE)

## Beschreibung

Die vorliegende Erfindung betrifft das Makrolid-Antibiotikum Swalpamycin, ein mikrobiologisches Verfahren zu seiner Herstellung, mit Hilfe des Mikroorganismus-Stammes Streptomyces species Y-84,30967 (DSM 3740) und seine Verwendung zur Herstellung von Arzneimitteln und Tierfutterzusatz.

Die Streptomyces-Art, Kulturnummer HIL Y-84,30967, im folgenden als Str. sp. Y-84,30967 bezeichnet, wurde aus einer bei Pune, Maharashtra (Indien) gesammelten Bodenprobe isoliert. Varianten und Mutanten dieses Stammes sind in bekannter Weise unter Verwendung eines Mutagens wie N-Methyl-N-nitro-N'-nitrosoguanidin oder durch ultraviolettes Licht erhältlich. Der Mikroorganismus Str. sp. Y-84,30967 gehört zur Ordnung Actinomycetales, Familie Streptomycetaceae und Gattung Streptomyces. Von einigen Streptomyces-Arten ist es bekannt, daß sie Antibiotika vom Makrolidtyp produzieren, und diese sind in der Literatur beschrieben (T. Osono, Y. Oka, S. Watanabe, Y. Numazaki, K. Moriyama, H. Ishida, K. Suzuke, Y. Okami und H. Umezawa: J. Antibiotics 20, 174 (1967); S. Omura, Y. Hironaki und T. Hata: J. Antibiotics 23, 511 (1970); I. Haupt, H. Fricke, J. Cerna und I. Rychlik: J. Antibiotics 29, 1314 (1976); S. Omura und H. Tanaka in "Macrolide Antibiotics [Makrolidantibiotika]", S. 3-35, S. Omura (Herausgeber), Academic Press, Orlando, Florida, USA (1984).

Str. sp. Y-84,30967 wird als ein neuer Stamm angesehen, da er sich in einigen seiner morphologischen, Züchtungs- und physiologischen Eigenschaften von den bekannten Stämmen unterscheidet, wie aus der nachfolgenden Beschreibung ersichtlich ist. Ein weiterer Grund dafür ist, daß er ein neues, hier als Swalpamycin bezeichnetes Makrolidantibiotikum und ein neues, Swalpanolid genanntes Makrolidaglykon produziert, wie in der nachfolgenden Beschreibung angegeben.

Makrolidantibiotika sind in der Medizin als antimikrobielle Mittel gegen ein breites Spektrum grampositiver Bakterien und Mycoplasmen von Bedeutung. Bisher wurden verschiedene Makrolidantibiotika beschrieben, z.B. Erythromycin, Leukomycin, Oleandomycin, Spiramycin und Midecamycin, die klinisch eingesetzt werden, sowie Tylosin, das in der Tierpraxis Anwendung findet.

Makrolide sind in einer Anzahl Übersichtsartikeln beschrieben (Z. Vankek und J. Majer in "Antibiotics", Band 2, S. 154-188; D. Gottlieb und P.D. Shaw (Herausgeber), Springer, New York (1967); D. Vasquez in "Antibiotics", Band 3, S. 459-479; J.W. Corcoran und F.E. Hahn (Herausgeber), Springer, New York; J. Berdy in "CRC Handbook of Antibiotic Compounds", Band 2, S, 39-156, CRC Press, Boca Raton, Florida (1980)).

Weiter findet sich eine Zusamenfassung der Makrolidantibiotika in "Index of Antibiotics from Actinomycetes", Hamao Umezawa (Hauptherausgeber), Band I, S. 114, 123,135, 171, 172, 207, 223, 275, 373, 382-384, 604-606, 640-641 und 669, Univ. Park Press, State College, Pennsylvania, USA; (1967), und Band II, S. 254, 255, 287-289, 305, 357-362, 481, 483, 508, 509, 523-537, 549-550, 573-579, 630-635, 639-641, 809-823 und 1013-1014, Univ. Park Press, Baltimore, USA, (1978).

Das von S. Omura herausgegebene und bei Academic Press, Orlando, Florida, USA, (1984), verlegte Buch "Macrolide Antibiotics - Chemistry, Biology and Practice" enthält eine umfassende Übersicht der Makrolidantibiotika, ihrer Biosynthese, chemische Derivatisierung, chemischen Synthese und ihrer Rolle in der Klinik und Veterinärpraxis.

Die Makrolidantibiotika werden nach der Größe des das Aglykon bildenden makrocyclischen Lactonrings in 3 Hauptgruppen eingeteilt (A.K. Mallams in "Macrolides", S. 156, M. Grayson (Herausgeber), Wiley, New York, (1982)). Die erste Gruppe stellt die Methymycin, Neomethymycin und YC-17 umfassende Gruppe 12-gliedriger Makrolide dar. Die zweite Gruppe besteht aus den 14-gliedrigen Makroliden, deren wichtige Mitglieder Erythromycin, Oleandomycin, Lankamycin, Kujimycin, Megalomycin, Pikromycin und Narbomycin sind. Bei der dritten Gruppe handelt es sich um die 16-gliedrigen Makrolide, die man unterteilen kann in

a) die basischen, mindestens einen Aminozuckerrest enthaltenden Makrolidantibiotika, wobei diese Untergruppe eine große Anzahl Antibiotika umfaßt, zu denen als Vertreter und wissenschaftlich wichtige Verbindungen Angolamycin, Leukomycine, Spiramycine, Maridomycine, Carbomycine, Cirramycine, Rosamycin, Macinamycine, M-4365 $G_1$ und $G_2$ sowie Tylosin zählen;

b) die neutralen, keine Aminozucker enthaltenden Makrolidantibiotika.

Als Mitglieder dieser Untergruppe werden Chalcomycin (P.W.K. Woo u.a., J. Amer. Chem. Soc. 84, 880; 1512; 1066 (1962)), Neutramycin (D.V. Lefemine u.a., Antimicrob. Agents Chemother., s. 41 (1961)); Aldgamycine (M.P. Kunstmann u.a., Antimicrob. Agents Chemother., S. 87 (1964)) und einige Mycinamycine (K. Kinoshita u.a.: J. Antibiotics, 38, 522-526 (1985)) angeführt. Wie aus der nachfolgenden näheren Beschreibung der Erfindung ersichtlich, gehört das erfindungsgemäße Swalpamycin zu dieser neutralen Makroliduntergruppe, unterscheidet sich aber von Chalcomycin, Neutramycin, den Aldgamycinen und Mycinamycinen, wie aus dem HPLC-Chromatogramm (siehe Fig. 1), physikalisch-chemischen und

spektroskopischen Eigenschaften (siehe Fig. 2-5) erkennbar ist.

Gegenstand der vorliegenden Erfindung ist daher das Makrolidantibiotikum Swalpamycin der Formel I

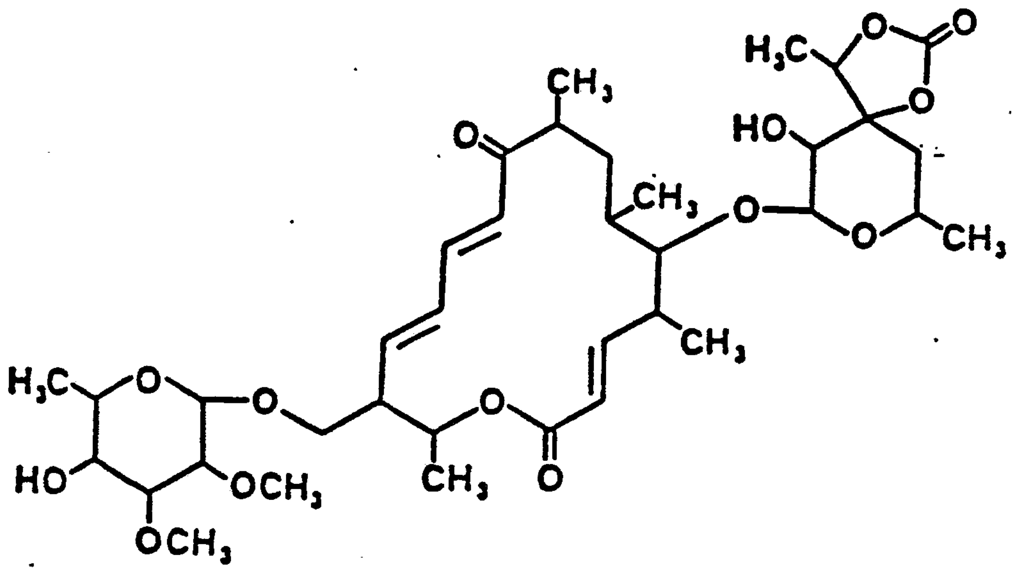

I

sowie das Makrolid-Aglykon Swalpanolid der Formel II

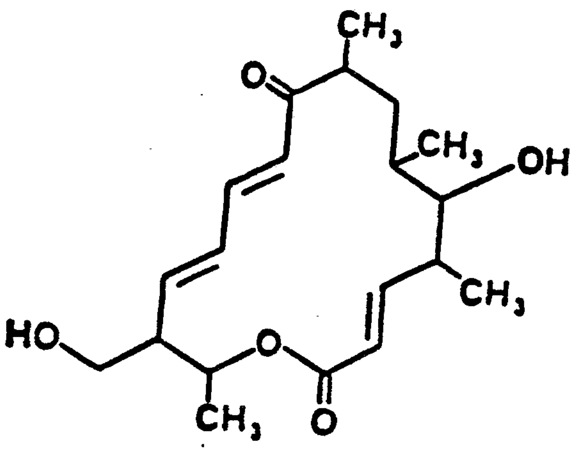

II

Gegenstand dieser Erfindung ist ferner ein Verfahren zur Herstellung des Makrolidantibiotikums Swalpamycin der Formel I und des Makrolidaglykons Swalpanolid der Formel II sowie von Chalcomycin. Das Verfahren ist dadurch gekennzeichnet, daß man Str. sp. Y-84,30967 (DSM 3740) unter aeroben Bedingungen bei einer Temperatur zwischen 18° und 37° in einem wäßrigen, assimilierbaren Kohlenstoff- und Stickstoffquellen sowie lebenswichtige Mineralsalze enthaltenden Nährmedium bei einem pH zwischen 6 und 9 züchtet, bis das Medium eine wesentliche antiobitische Aktivität aufweist, und das Fermentationsprodukt aus dem Medium gewinnt.

Das erfindungsgemäße neue Antibiotikum ist gegen eine Anzahl pathogener Mikroorganismen einschließlich grampositiver Bakterien und Mycoplasmen aktiv und demgemäß in der Human- und Tiermedizin verwendbar. Insbesondere eignet sich Swalpamycin als therapeutisches Arzneimittel zur Behandlung von durch grampositive Bakterien und Mycoplasmen, beispielsweise Staphylococcus aureus, Streptococcus pyogenes, Diplococcus pneumoniae, Legionella pneumoniae, Rickettsia-Stämme, Spirochaeta, Toxoplasma, Clostridium tetani, Bacteroides fragilis, Mycobacterium kansasi, Mycoplasma gallinarum, Mycoplasma pneumoniae, Chlamydia trachomatis usw., verursachten Infektionskrankheiten. Ferner ist das neue erfindungsgemäße Antibiotikum gegen Stämme aktiv, die gegen unterschiedliche Antibiotika einschließlich Erythromycin resistent sind. Es ist auch als Tierfutterzusatz verwendbar. Swalpamycin läßt sich in jeglicher biologisch verfügbarer Form entweder für sich oder mit Chalcomycin kombiniert einsetzen.

Der Stamm Streptomyces sp. Y-84,30967 wird aus einer Bodenprobe durch Züchten bei einem pH von 6,5 bis 8,5 in einem Nährmedium gewonnen, das aus Kohlenstoff- und Stickstoffquellen, anorganischen

Nährsalzen und Verfestigungsmitteln, besteht. Als Kohlenstoffquellen kommen Glucose, Stärke, Dextrin, Glycerin, Rohrzucker und Melasse in Frage. Die in Betracht kommenden Stickstoffquellen sind Pepton, Hefeextrakt, Rindfleischextrakt, Malzextrakt und Casein. Das Verfestigungsmittel kann beispielsweise Agar sein. Zu anorganischen Nährsalzen zählen Salze des Natriums, Kaliums, Magnesiums, Calciums, Phosphors und Schwefels.

Der erfindungsgemäß Mikroorganismus verlängert farblose Luftmycelien aus verzweigten Substratmycelien. Sporenketten werden in Spiralen über den Luftmycelien gebildet. Weder Verwirbelungen noch Sporenschläuche sind zu beobachten. Die Züchtungseigenschaften des Mikroorganismus auf verschiedenen Agarmedien lassen sich wie folgt beschreiben:

1. Hefeextrakt/Malzextrakt-Agar
Wachstum : reichlich
Luftmycel : reichlich, weiß bis rosagrau, pulverig
Unterseite : hellbraun
lösliches Pigment : negativ

2. Hafermehl-Agar
Wachstum : reichlich
Luftmycel : reichlich, weiß bis grau bis hellbraun, pulverig
Unterseite : hellgelb
lösliches Pigment : negativ

3. Anorganische Salze/Stärke-Agar
Wachstum : reichlich
Luftmycel : reichlich, grau bis bräunlich grau, pulverig
Unterseite : hellbraun
lösliches Pigment : negativ

4. Glycerin/Asparagin-Agar
Wachstum : gut
Luftmycel : schwach, weiß, pulverig
Unterseite : hellgelb
lösliches Pigment : negativ

5. Pepton/Hefeextrakt/Eisen-Agar
Wachstum : mäßig
Luftmycel : negativ
Unterseite : dunkelbraun bis schwarz
lösliches Pigment : bräunlich schwarz

6. Tyrosin-Agar
Wachstum : gut
Luftmycel : gut, weiß bis rosagrau, pulverig
Unterseite : bräunlich schwarz
lösliches Pigment : hellbraun

7. Rohrzucker/Nitrat-Agar
Wachstum : schwach
Luftmycel : spärlich, weiß, pulverig
Unterseite : hellbraun
lösliches Pigment : negativ

8. Glucose/Asparagin-Agar
Wachstum : mäßig
Luftmycel : schwach, weiß bis graubraun, pulverig
Unterseite : hellgelb
lösliches Pigment : negativ

9. Nähr-Agar
Wachstum : mäßig
Luftmycel : negativ
Unterseite : hellbraun
lösliches Pigment : negativ

Der für das Wachstum des erfindungsgemäßen Mikroorganismus optimale Temperaturbereich liegt bei 25 °C bis 35 °C. Der Mikroorganismus verflüssigt Gelatine in einem Glucose/Pepton/Gelatinemedium, hydrolysiert Stärke in einem Stärke/anorganische Salze-Agar und bringt Magermilch zum Gerinnen.

Die Bildung eines Melanoidpigments ist in Tyrosinagar, Pepton/Hefeextrakt/Eisen-Agar und

Trypton/Hefeextraktbrühe zu beobachten.

Das Assimilationsbild dieses Mikroorganismus für Kohlenstoffquellen ist wie folgt (in Medium nach Pridham-Gottlieb):

| | |
|---|---|
| Positive: | D-Glucose, L-Arabinose, D-Xylose, I-Inosit, D-Mannit, D-Fructose, Raffinose, Galaktose, Maltose, Cellobiose, Na-Glutamat, Mannose, Lactose. |
| Schwach positiv: | Rohrzucker |
| Negativ: | Rhamnose, Cellulose, Salicin, Dulcit. |

Hinsichtlich der Bildung der bekannten 16-gliedrigen neutralen Makrolidantibiotika wie Chalcomycin, Neutramycin, den Aldgamycinen, Mycinamycinen und Bandamycinen sind die folgenden bekannten Mikroorganismen mit Str. sp. Y-84,30967 zu vergleichen: Streptomyces bikiniensis, Streptomyces albogriseolus, Streptomyces rimosus, Streptomyces goshikiensis und Streptomyces lavendulae.

Mycinamycine werden bekanntlich von Micromonospora griseorubida sp. nov. gebildet.

Die veröffentlichten Angaben über die Züchtungs- und physiologischen Eigenschaften dieser bekannten Mikroorganismen zeigen deutliche Unterschiede zwischen dem erfindungsgemäßen Mikroorganismus und den oben erwähnten bekannten Mikroorganismen. Beispielsweise gehören Streptomyces lavedulae und Streptomyces goshikiensis zur roten Serie und Streptomyces rimosus zur weißen Serie, während der erfindungsgemäße Mikroorganismus in der grauen Serie einzustufen ist. Streptomyces albogriseolus läßt sich von Streptomyces sp. Y-84,30967 auf Grund der Bildung von melanoidem Pigment differenzieren. Der erfindungsgemäße Mikroorganismus zeigt deutliche Unterschiede zu Streptomyces bikiniensis im Assimilationsbild für Kohlenstoffquellen. Ferner bildet der erfindungsgemäße Mikroorganismus bei der Fermentation Chalcomycin und ein als Swalpamycin bezeichnetes neues Makrolidantibiotikum, der Formel I das ein neues 16-gliedriges Makrolidaglykon enthält. Auf Grundlage der obigen Beobachtungen wird geschlossen, daß der erfindungsgemäße Mikroorganismus eine neue Art Streptomyces darstellt.

Der Mikroorganismus-Stamm wurde am 20. Mai 1986 bei der "Deutsche Sammlung von Mikroorganismen" (D-3400 Göttingen) unter der Eingangsnummer DSM 3740 hinterlegt.

Est ist für den Fachmann klar, daß diese Erfindung nicht auf den speziellen, oben definierten Mikroorganismus beschränkt ist, sondern alle solche spontanen und künstlichen, von besagtem Mikroorganismus abgeleiteten Mutanten einschließt, die dazu befähigt sind, das neue Antibiotikum Swalpamycin bzw. das neue Makrolidaglykon Swalpanolid zu bilden.

Das Verfahren zur Herstellung von zur Klasse der Makrolidantibiotika gehörenden Verbindungen, ist dadurch gekennzeichnet, daß man Str. sp. Y-84,30967 (DSM 3740) bei einem pH zwischen 6,0 und 7,0 und einer Temperatur zwischen 20 und 40 °C unter aeroben Bedingungen in einem Kohlenstoff- und Stickstoffquellen, anorganische Nährsalze und Spurenelemente enthaltenden Nährmedium kultiviert und die Verbindungen aus den Kulturbrühen und dem Mycel in bekannter, im folgenden beschriebener Weise gewinnt.

Für das Nährmedium zur Herstellung der neuen Antibiotika kommen als Kohlenstoffquellen Glucose, Stärke, Dextrin, Glycerin, Rohrzucker, Melasse und Öl, als Stickstoffquellen Sojabohnenmehl, Hefeextrakt, Rindfleischextrakt, Malzextrakt, Maisquellwasser, Peptone und Casein, als anorganische Nährsalze Natriumchlorid, Magnesiumsulfat, Ammoniumsulfat und Calciumcarbonat infrage. Eisen, Mangan, Kupfer, Zink und Kobalt können als Spurenelemente verwendet werden.

Vorzugsweise wird Str. sp. Y-84, 30967 (DSM 3740) bei 25-30 °C, besonders bevorzugt bei 27 °C und pH 6,5 kultiviert. Die Fermentation wird nach 40-45 Stunden abgebrochen, wobei man die höchsten Ausbeuten der Verbindungen erhält. Vorzugsweise erfolgt die Fermentation als Submersfermentation. Der Fortschritt der Fermentation und die Bildung der antibiotischen Makrolidverbindungen läßt sich durch die antibakerielle Wirksamkeit der Kulturflüssigkeit und des Mycels gegen Staphylococcus aureus 209 P verfolgen.

Swalpamycin kann man aus dem Filtrat bei pH 6,0-7,5, vorzugsweise pH 6,5, als Gemisch mit Chalcomycin unter Anwendung herkömmlicher Methoden wie Extraktion mit nicht mit Wasser mischbaren Lösungsmitteln wie Chloroform, Methylenchlorid, n-Butanol, Essigester oder Butylacetat gewinnen, wobei Essigester als das mit Wasser nicht mischbare Lösungsmittel bevorzugt wird.

Der antibiotische Komplex kann aus dem Mycel oder der Gesamtkulturbrühe durch Extraktion des Mycelkuchens mit einem organischen Lösungsmittel z.B. einem Keton vorzugsweise Aceton, gewonnen werden. Dabei wird der Acetonextrakt eingeengt, die wäßrige Schicht auf pH 6,0-7,5, vorzugsweise pH 6,5, eingestellt und mit nicht mit Wasser mischbaren organischen Lösungsmitteln wie Chloroform, Methylenchlorid, n-Butanol, Essigester oder Butylacetat extrahiert, wobei Essigester als Lösungsmittel bevorzugt wird. Die Essigesterextrakte des Mycels und des Kulturfiltrats kann man vereinigen oder getrennt aufarbeiten.

Eine weitere Methode zur Gewinnung von Swalpamycin aus der Kulturbrühe beruht auf Adsorption. Dazu wendet man auf die flüssige Substanz, beispielsweise das Kulturfiltrat oder den die erfindungsgemäße Verbindung enthaltenden Lösungsmittelextrakt Säulenchromatographie oder Flüssigkeitschromatographie

usw. unter Verwendung geeigneter Adsorptionsmittel wie Aktivkohle, ®Diaion HP-20, ®XAD 4, Aluminiumoxid, Kieselgel oder ®Sephadex LH-20 an. Die erfindungsgemäße Verbindung wird aus den Adsorptionsmitteln unter Verwendung geeigneter mobiler Phasen wie Chloroform, Methanol oder Aceton für sich oder in Kombination miteinander oder mit Wasser eluiert, und die Eluate werden zur Trockne eingedampft.

Aus dem oben erwähnten Swalpamycin und Chalcomycin enthaltenden Lösungsmittelextrakten bzw. dem eingeengten Eluat können die erfindungsgemäßen Verbindungen nach verschiedenen Methoden gewonnen und gereinigt werden. Beispielsweise kann man zur Gewinnung und Reinigung zweckmäßig Adsorptions- und Elutionsverfahren mit Aktivkohle, ®Amberlit, ®XAD-4 und 7 (Röhm und Haas Co.) oder ®Diaion HP-20 (Mitsubishi Chemical Industries), Gelfiltration mit ®Sephadex LH-20 (Pharmacia Fine Chemicals AB) und gleichwertigen Produkten sowie Adsorptionschromatographie auf Aluminiumoxid und Kieselgel usw. kombinieren. Ferner kommen Dünnschichtchromatographie, Mitteldruck- und Hochleistungsflüssigkeitschromatographie mit geeigneten Adsorptionsmitteln wie Kieselgel und silanisiertem Kieselgel C18 mit geeigneten Lösungsmittelsystemen in Frage. Weiterhin kann Gegenstromchromatographie mit einem bestimmten Lösungsmittelsystem den besagten Zweck erfüllen.

Swalpamycin ist ein farbloses amorphes Pulver, das in Methanol, Äthanol, n-Propanol, Isopropanol, n-Butanol, Essigester, n-Butylacetat, Aceton, Äthylmethylketon, Methylisobutylketon, Methylenchlorid, Chloroform, Dimethylformamid und Dimethylsulfoxyd löslich ist. Swalpamycin ist schwer löslich oder unlöslich in Hexan, Petroläther (40-60°) und Wasser.

In den unten angeführten Dünnschichtchromatographiesystemen (DC) zeigen Swalpamycin und Chalcomycin die folgenden Werte:
DC-Platte: Fertigplatte Kieselgel (Artikel Nr. 5554 von E. Merck, Darmstadt)

| Rf | in $MeOH:CHCl_3$ (7 : 93) | $MeOH:CHCl_3$ (1 : 9) | Essigester |
|---|---|---|---|
| Swalpamycin | 0,5 | 0,6 | 0,7 |
| Chalcomycin | 0,42 | 0,53 | 0,46 |

Bei der in Fig. 1 gezeigten analytischen Hochleistungsflüssigkeitschromatographie (HPLC) zeigen Swalpamycin und Chalcomycin die folgenden Retentionszeiten:

Säulenfüllung : ®Lichrosorb RP18
Durchflußgeschwindigkeit : 1 ml/min
Nachweis : bei 216 nm
Lösungsmittel : $MeOH:H_2O$ (7:3)

Retentionszeit
Swalpamycin : 5,7 min
Chalcomycin : 5,0 min

Swalpamycin schmilzt bei 126-129 °C und besitzt einen optischen Drehwert von $[\alpha]_D^{20}$ = -3,8 ° (c = 0,21 in $CHCl_3$).

Die spektroskopischen Daten für Swalpamycin sind wie unten angeführt:

1. UV $\lambda_{max}$ in Methanol: siehe beigefügte Fig. 2. Zur Bestimmung der UV-Absorptionsmaxima des Swalpamycins wurde eine Lösung mit einer Konzentration von 0,2 g/l verwendet. Das Absorptionsspektrum wurde im Bereich 200 bis 800 nm gemessen.
2. Das IR-Spektrum (KBr-Tablette) wurde mit einem IR-Spektrometer Perkin Elmer P.E.521 bestimmt - siehe beigefügte Fig. 3.

Die $^1H$- und $^{13}C$-Kernresonanzspektren wurden in $CDCl_3$ mit einem Fouriertransform-Kernresonanzspektrometer Jeol Fx-90 bei 90 MHz bzw. 22,5 MHz mit $Me_4Si$ als innerem Standard bestimmt.

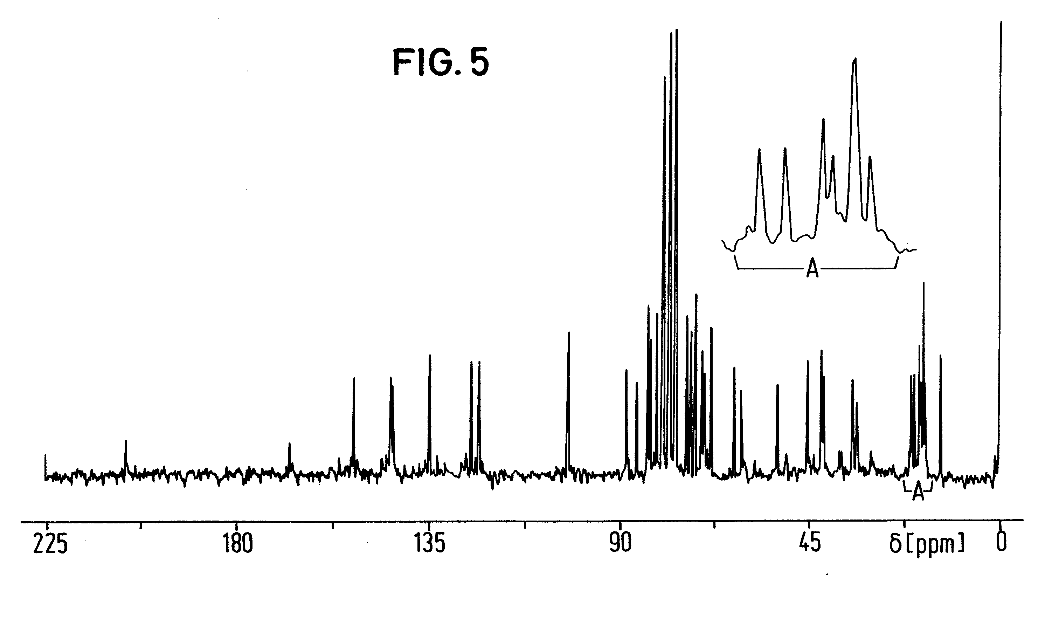

3. $^1H$-NMR-Spektrum - siehe beigefügte Fig. 4.
4. $^{13}C$-NMR-Spektrum - siehe beigefügte Fig. 5.
5. Die massenspektrographischen Untersuchungen wurden am silylierten Derivat des Swalpamycins mit Elektronenstoßionisierung durchgeführt, und die Summenformel für jeden Peak, einschließlich des $M^+$-Peaks wurde durch Hochauflösungsmassenmessungen bestimmt.

Dabei ergaben sich das Molekulargewicht und die Summenformel wie folgt:

Swalpamycin - Molekulargewicht : 724
- Summenformel : $C_{37}H_{56}O_{14}$

Aus den oben beschriebenen, zur Verfügung stehenden Daten wird auf die in den oben angeführten Formeln I und II gezeigten Konstitutionen für Swalpamycin bzw. Swalpanolid geschlossen.

Swalpamycin weist bei Agarplattentests im Laboratorium ein antimikrobielles Spektrum gegen Bakterien, Mycoplasma und Aktinomyceten auf. In vitro besitzt es eine Wirkung gegen empfindliche und resistente

Stämme von Staphylococcus aureus und Streptococcus faecalis sowie Stämme von Bacillus subtilis, Sarcina lutea, Streptococcus pyogenes, Micrococcus luteus, Mycoplasma gallinarum and Mycoplasma pulmonis. Die minimale Hemmkonzentration (MHK) von Swalpamycin gegen verschiedene Mikroorganismen wurde bestimmt. Die Ergebnisse finden sich in der nachfolgenden Tabelle I:

Tabelle I

| MHK von Swalpamycin | | |
|---|---|---|
| Nr | Testorganismus | Swalpamycin |
| 1. | S. aureus 209P | 6,3 |
| 2. | S. aureus 20424 $Mac^R$ | 50 |
| 3. | S. aureus 3066 $Meth.^R$ | 3,2 |
| 4. | S. aureus R 85,$Em^R$ | 12,5 |
| 5. | S. aureus R85/M, $Em^R$ | > 50 |
| 6. | S. aureus 712 $Meth^R$ | 12,5 |
| 7. | S. aureus 789 $Meth.^R$ | > 50 |
| 8. | Strept. faecalis UD 86 | > 50 |
| 9. | Strept. faecalis Eder:$Mac^R$ | > 50 |
| 10. | Micrococcus luteus | 1,6 |
| 11. | Sarcina lutea | 0,2 |
| 12. | Bacillus subtilis | 0,1 |
| 13. | S. aureus MLS 11,$Em^R$ | 25 |
| 14. | S. aureus MLS 14,$Em^R$ | 25 |
| 15. | S. aureus MLS 16,$Em^R$ | 12,5 |
| 16. | Candida albicans | > 50 |
| 17. | E. coli 9632 | > 50 |
| 18. | E. Coli ESS 2231 | > 50 |
| 19. | Ps. aeruginosa M 35 | > 50 |
| 20. | Pr. vulgaris | > 50 |
| 21. | Ent. cloacae | > 50 |
| 22. | Prov. stuartii | > 50 |
| 23. | Cit. freundii | > 50 |
| * Mac = Macrolid, Em = Erythromycin, Meth = Methicillin | | |

Swalpamycin ist somit gegen grampositive Bakterien einschließlich Erythromycin-resistenter Stämme und Mycoplasma wirksam und kann zur Behandlung von durch die obigen pathogenen Bakterien verursachten Infektionen verwendet werden.

Die Erfindung betrifft somit auch Arzneipräparate zur Behandlung von mikrobiellen Infektionen, die durch einen Gehalt an der Verbindung der Formel I charakterisiert sind.

Die erfindungsgemäße Verbindung kann auch in Kombination mit anderen Wirstoffen, beispielsweise aus der Reihe der Penicilline, Cephalosporine oder Aminoglykoside zur Anwendung kommen.

Die Verbindung der Formel I und ihre physiologisch verträglichen Säureadditionssalze können oral, intramuskulär oder intravenös verabfolgt werden. Arzneipräparate, die Swalpamycin als Wirkstoff enthalten, können hergestellt werden, indem man die Verbindung der Formel I mit einem oder mehreren pharmakologisch verträglichen Trägerstoffen oder Verdünnungsmitteln, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Für die parenterale Applikation kommen vorzugsweise Suspensionen oder Lösungen in Wasser in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindung der Formel I oder ihrer physiologisch verträglichen Säureadditionssalze liegen bei etwa 0,4 bis 20 g/Tag, vorzugsweise bei 0,5 bis 4 g/Tag für einen Erwachsenen von etwa 60 kg Körpergewicht.

Es können Einzel- oder im allgemeinen Mehrfachdosen verabreicht werden, wobei die Einzeldosis den

Wirkstoff in einer Menge von etwa 50 bis 1000 mg, vorzugsweise von etwa 100 bis 500 mg enthalten kann.
Die nachfolgenden Beispiele erläutern die Erfindung:

Beispiel 1
Isolierung von Streptomyces sp. Y-84,30967 aus Boden

a) Herstellung der Isolierungsnährmedien

| Medium 1: | |
|---|---|
| Glucose | 1,0 g |
| Glycerin | 1,0 g |
| L-Arginin | 0,3 g |
| $K_2HPO_4$ | 0,3 g |
| $MgSO_4.7H_2O$ | 0,2 g |
| NaCl | 0,3 g |
| Hefeextrakt | 2,0 g |
| $Fe_2(SO_4)_3$ | 10 mg |
| $CuSO_4.5H_2O$ | 1 mg |
| $ZnSO_4.7H_2O$ | 1 mg |
| $MnSO_4.7H_2O$ | 1 mg |
| Agar | 15,0 g |
| Destilliertes Wasser | 1 Liter |
| pH | 6,5 |

| Medium 2: | |
|---|---|
| Glucose | 2,0 g |
| L-Asparagin | 1,0 g |
| $K_2HPO_4$ | 0,5 g |
| $MgSO_4.7H_2O$ | 0,5 g |
| Bodenextrakt | 200 ml |
| Agar | 15,0 g |
| Destilliertes Wasser | 800 ml |
| pH | 8,0 |

| Medium 3: | |
|---|---|
| Stärke | 10,0 g |
| Casein | 0,3 g |
| $KNO_3$ | 2,0 g |
| NaCl | 2,0 g |
| $K_2HPO_4$ | 2,0 g |
| $MgSO_4.7H_2O$ | 0,05 g |
| $CaCO_3$ | 0,02 g |
| $FeSO_4$ | 0,01 g |
| Agar | 15,0 g |
| Destilliertes Wasser | 1 Liter |
| pH | 7,2-7,5 |

Die Medien wurden 1/2 Stunde lang bei 121 °C sterilisiert.

b) Herstellung der Bodensuspension

Man suspendiert ein Gramm Boden in destilliertem Wasser unter gutem Schütteln. Man läßt den Boden sich absetzen und verwendet die überstehende Flüssigkeit jeweils zur Impfung eines der oben erwähnten Isoliermedien.

c) Impfung des Isoliermediums

Man beimpft 50 ml eine der oben erwähnten Isoliernährmedien enthaltende Petrischalen mit je ein ml Bodensuspension.

d) Isolierung von Streptomyces sp. Y-84,30967

Die beimpfte Petrischale wird zwei Wochen lang bei 30 °C inkubiert und Streptomyces sp. Y-84,30967 aus den wachsenden Mikroorganismen isoliert.

Beispiel 2

Züchtung von Streptomyces sp. Y-84, 30967 zur fermentativen Herstellung von Swalpamycin

Streptomyces sp. Y-84, 30967 wird auf Hefe-Malzagar der folgenden Zusammensetzung gehalten:

| | |
|---|---|
| Malzextrakt | 10,0 g |
| Hefeextrakt | 4,0 g |
| Glucose | 4,0 g |
| Agar | 15,0 g |
| Destilliertes Wasser | 1 Liter |
| pH | 7,0 |

Das Medium wird auf Reagenzgläser verteilt und 30 Minuten lang bei 121 °C sterilisiert. Zur Schrägagarpräparierung werden die Gläser in Schrägstellung abgekühlt. Ein Schrägagar wird jeweils mit der Kultur beimpft und 10-15 Tage lang bei 28 °C inkubiert, zu welcher Zeit man gutes Wachstum und Sporenbildung beobachtet. Eine Sporensuspension aus einem Schrägagar in destilliertem Wasser wird dazu verwendet, 5 je 100 ml Impfkulturmedium enthaltende 500 ml-Erlenmeyerkolben bzw. eine 1 l desselben Impfkulturmediums enthaltende 5 l-Saugflasche zu beimpfen.

| Zusammensetzung des Impfkulturmediums | |
|---|---|
| Glucose | 15,0 g |
| Sojabohnenmehl | 15,0 g |
| Maisquellwasser | 5,0 g |
| $CaCO_3$ | 2,0 g |
| NaCl | 5,0 g |
| Destilliertes Wasser | 1 Liter |
| pH | 6,5 |

Das obige Medium wird in Mengen von je 100 ml über 500 ml-Erlenmeyerkolben oder je 1 l in 5 l-Saugflaschen verteilt und 30 Minuten lang bei 121 °C sterilisiert. Die Kolben/Flaschen werden abgekühlt, mit der Sporensuspension beimpft und 72 Stunden lang bei 27 °C (± 1 °C) auf einer rotierenden Schüttelmaschine mit 38 mm Ausschlag bei 240 U.p.M. geschüttelt. Das so gewachsene Produkt wird dazu verwendet, zwei je 10 l 8-10 vol.%-iges Impfkulturmedium enthaltende 15 l-Glasfermentationsgefäße zur Herstellung der zweiten Impfkulturstufe zu beimpfen. Die Fermentation wird bei 27 °C (± 1 °C) unter Rühren bei 180-200 U.p.M. und einer Belüftungsmenge von 6-7 Liter/Min. über einen Zeitraum von 24 Stunden durchgeführt. Die so erhaltene, gut gewachsene zweite Impfkulturstufe wird zur Beimpfung des Produktionsmediums verwendet.

| Zusammensetzung des Produktionsmediums | |
|---|---|
| Glucose | 15,0 g |
| lösliche Stärke | 20,0 g |
| Sojabohnenmehl | 3,0 g |
| Pepton | 3,0 g |
| $CaCO_3$ | 2,0 g |
| NaCl | 2,0 g |
| Maisquellwasser | 2,0 g |
| $(NH_4)_2SO_4$ | 0,5 g |
| Destilliertes Wasser | 1 Liter |
| pH | 6,5 |

Als Entschäumer wird dem Inhalt des Fermentationsgefäßes 0,025 % Desmophen® zugesetzt.

Man gibt 280 l des obigen Mediums in ein 390 l-Fermentationsgefäß und sterilisiert mittels indirektem und direktem Dampf 28 Minuten lang bei 121 °C. Das Fermentationsgefäß wird abgekühlt und mit der zweiten Impfkulturstufe (7 Vol.-%) beimpft. Die Fermentation erfolgt bei 27 °C (± 1 °C) unter Rühren bei 100-200 U.p.M. für einen Zeitraum von 40-45 Stunden. Die Belüftungsgeschwindigkeit beträgt 170 Liter pro Minute. Bei Beendigung der Fermentation nach 40-45 Stunden beträgt der Durchmesser der Hemmzone gegen Staphylococcus aureus 209P 16 mm bei der Prüfung des Kulturfiltrats nach der Agarnäpfchenmethode (6 mm Durchmesser) bei einem pH der Kulturflüssigkeit im Bereich von 6,4 bis 6,8.

Das abgeschiedene Zellvolumen beträgt 28-30 Vol.-%.

Die geerntete, den Antibiotikumkomplex enthaltende Kulturbrühe wird zur Abtrennung des Mycels zentrifugiert, und die Kulturflüssigkeit wird wie in Beispiel 6 beschrieben weiter verarbeitet.

Beispiel 3

Kultivierung von Streptomyces sp. Y-84,30967 zur fermentativen Herstellung von Swalpamycin

Man verfährt wie in Beispiel 2 mit den folgenden Unterschieden:

| Zusammensetzung des Agarmediums | |
|---|---|
| Stärke (löslich) | 10,0 g |
| $K_2HPO_4$ | 1,0 g |
| $MgSO_4.7H_2O$ | 1,0 g |
| NaCl | 1,0 g |
| $(NH_4)_2SO_4$ | 2,0 g |
| $CaCO_3$ | 2,0 g |
| $FeSO_4.7H_2O$ | 0,1 mg |
| $MnCl_2.4H_2O$ | 0,1 mg |
| $ZnSO_4.7H_2O$ | 0,1 mg |
| Agar | 15,0 g |
| Destilliertes Wasser | 1 Liter |
| pH | 7,2 |

| Zusammensetzung des Produktionsmediums | |
|---|---|
| Glucose | 20,0 g |
| Sojabohnenmehl | 10,0 g |
| $CaCO_3$ | 0,2 g |
| $CoCl_2.6H_2O$ | 0,1 g |
| Destilliertes Wasser | 1 Liter |
| pH | 7,0 |

100 l des obigen Mediums werden in einem 150 l-Fermentationsgefäß vorgelegt und mittels direktem und

indirektem Dampf 28 Minuten lang bei 121 °C sterilisiert.

Das Fermentationsgefäß wird abgekühlt und mit der zweiten Impfkulturstufe (9 Vol.-%) beimpft. Die Fermentation erfolgt bei 27 °C (± 1 °C) unter Rühren bei 80-90 U.p.M. mit einer Belüftungsgeschwindigkeit von 60-70 Litern pro Minute. Bei Beendigung der Fermentation nach 40-43 Stunden beträgt der pH der Kulturbrühe 6,45 und der Durchmesser der Hemmzone gegen Staphylococcus aureus 209P 15 mm bei der Prüfung des Kulturfiltrats nach der Agarnäpfchenmethode (6 mm Durchmeser). Das abgeschiedene Zellvolumen beträgt 18-24 Vol.-%. Die Kulturbrühe wird wie in Beispiel 7 verarbeitet.

Beispiel 4

Kultivierung von Steptomyces sp. Y-84,30967 zur Herstellung von Swalpamycin

Man verfährt wie in Beispiel 2 mit den folgenden Unterschieden:

| Zusammensetzung des Agarmediums | |
|---|---|
| Hafermehl | 20,0 g |
| Agar | 15,0 g |
| $FeSO_4.7H_2O$ | 0,1 g |
| $ZnSO_4.7H_2O$ | 0,1 mg |
| $MnCl_2.4H_2O$ | 0,1 mg |
| Destilliertes Wasser | 1 Liter |
| pH | 7,2 |

| Zusammensetzung des Impfkulturmediums | |
|---|---|
| Stärke | 30,0 g |
| Rohrzucker | 10,0 g |
| Glucose | 10,0 g |
| Sojapepton | 15,0 g |
| Maisquellwasser | 10,0 g |
| $K_2HPO_4$ | 3,0 g |
| NaCl | 1,0 g |
| $CaCO_3$ | 3,0 g |
| $CuSO_4.5H_2O$ | 7,0 mg |
| $FeSO_4.7H_2O$ | 1,0 mg |
| $MnCl_2.4H_2O$ | 8,0 mg |
| $ZnSO_4.7H_2O$ | 2,0 mg |
| Destilliertes Wasser | 1 Liter |
| pH | 7,2 |

| Zusammensetzung des Produktionsmediums | |
|---|---|
| Dextrin | 20,0 g |
| Sojabohnenmehl | 10,0 g |
| Hefeextrakt | 2,0 g |
| $FeSO_4.7H_2O$ | 0,1 g |
| Destilliertes Wasser | 1 Liter |
| pH | 6,5 |

Beim Abernten des Fermentationsgefäßes nach 49 Stunden beträgt der pH der Kulturbrühe 6,75 und das abgeschiedene Zellvolumen 22 Vol.-%. Der Durchmesser der Hemmzone gegen Staphylococcus aureus 209P ist 15 mm bei der Prüfung des Kulturfiltrats nach der Agarnäpfchenmethode (6mm).

Die geerntete Kulturbrühe wird wie in Beispiel 6 weiterverarbeitet.

Beispiel 5

Kultivierung von Streptomyces sp. Y-84,30967 zur fermentativen Herstellung von Swalpamycin

Man verfährt wie in Beispiel 3 mit den folgenden Unterschieden:

| Zusammensetzung des Produktionsmediums | |
|---|---|
| Pepton | 10,0 g |
| Rindfleischextrakt | 5,0 g |
| Glucose | 20,0 g |
| NaCl | 5,0 g |
| $CaCO_3$ | 3,0 g |
| Destilliertes Wasser | 1 Liter |
| pH | 6,5 |

Beim Abernten des Fermentationsgefäßes nach 46 Stunden beträgt der pH der Kulturbrühe 6,5 und das abgeschiedene Zellvolumen 27 Vol.-%, und das Kulturfiltrat zeigt 16 mm Aktivität (Durchmesser der Hemmzone) bei der Prüfung nach der Agarnäpfchenmethode (6 mm Durchmesser).

Die geernetete Kulturbrühe wird wie in Beispiel 7 weiterverarbeitet.

Beispiel 6

Isolierung und Reinigung des Swalpamycins

Die in Beispiel 2 erhaltene Fermentationsbrühe wird zur Trennung in Mycel und Kulturfiltrat zentrifugiert. Das dabei erhaltene Kulturfiltrat (232 l) wird nötigenfalls mit 2 n-HCl auf pH 6,5 eingestellt und zweimal mit je 120 l Essigester extrahiert. Die wäßrigen Schichten werden verworfen und die vereinigten Essigesterextrakte im Vakuum zur Trockne eingedampft. Die Menge des dabei erhaltenen rohen Extrakts beträgt 165 g. Das Mycel (14,5 kg) wird zweimal mit je 45 l Aceton extrahiert. Zur Entfernung des Acetons werden die vereinigten Extrakte im Vakuum eingedampft. Die so erhaltene wäßrige Schicht wird mit 2-nNaOH auf pH 6,5 eingestellt, mit 20 l Wasser versetzt und dreimal mit je 20 l Essigester extrahiert. Die wäßrigen Schichten werden verworfen und die vereinigten Extrakte im Vakuum zur Trockne eingedampft. Dabei erhält man ca. 110 g rohen Extrakt. Die rohen Extrakte aus dem Kulturfiltrat und dem Mycel werden vereinigt (275 g), in 1 l 10 %igem wäßrigen Methanol aufgelöst und viermal mit je 1 l Heptan weiterbehandelt. Die Heptanschicht wird verworfen und die wäßrige Schicht bei vermindertem Druck eingeengt. Nach Entfernung des Methanols extrahiert man die wäßrige Schicht zweimal mit je 500 ml Chloroform. Der so erhaltene Chloroformextrakt wird bei vermindertem Druck eingeengt, was 110 g der das Antibiotikumgemisch enthaltenden Verbindung liefert.

Diese 110 g werden zweimal nacheinander auf eine 3,5 kg Kieselgel (Siebgröße 300-400 mesh) enthaltende Säule aufgegeben und mit einem aus Chloroform:Methanol (97:3) bestehenden Lösungsmittelsystem eluiert. Auf diese Weise erhält man 6 g aktive Fraktion A und 80 g aktive Fraktion B. Die Fraktion B wird weiter durch wiederholte Kieselgelchromatographie (2,4 kg Kieselgel, Siebgröße 100-200 mesh) zu 9 g halbreinem Chalcomycin und weiteren aktiven Fraktionen gereinigt.

1 g halbreines Chalcomycin wird durch präparative Dünnschichtchromatographie (0,5 mm dicke Kieselgelplatten) zu 70 mg reinem Chalcomycin gereinigt. Das Chalcomycin wird durch seine physikalisch-chemischen Eigenschaften und spektroskopische Analyse identifiziert. Zur weiteren Reinigung wird die Fraktion A auf eine 600 g Kieselgel RP18 enthaltende Säule aufgegeben und mit einem aus Methanol:Wasser (7:3) bestehenden Lösungsmittelsystem eluiert, wobei man 85 mg halbreines Swalpamycin erhält. Dessen weitere Reinigung wird durch präparative Dünnschichtchromatographie (0,5 mm dicke Kieselgelplatten) in einem Gemisch aus Chloroform und Methanol (90:10) und nachfolgende präparative Hochleistungsflüssigkeitschromatographie auf einer ®$\mu$-Bondapak C18-Säule (Waters) mit Methanol:Wasser (7:3) als Eluiermittel bei einer Durchflußgeschwindigkeit von 1 ml/Minute unter Verwendung eines UV-Detektors bei 280 nm erreicht. Dabei erhält man 23 mg reines Swalpamycin.

Das Swalpamycin wird durch chemische Analyse und spektroskopische Methoden identifiziert.

Beispiel 7

Isolierung und Reinigung von Swalpamycin

Ungefähr 62 Liter der gemäß Beispiel 3 erhaltenen Fermentationsbrühe werden zur Trennung des Mycels und Kulturfiltrats zentrifugiert. Das Filtrat aus der Brühe wird zweimal mit je 30 Litern Essigester extrahiert. Die Essigesterextrakte werden vereinigt und im Rotationsverdampfer zur Trockne eigedampft, was 12 g rohen Extrakt liefert. Das Mycel wird zweimal mit je 6 Litern Aceton extrahiert. Die Acetonextrakte werden vereinigt, und nach Entfernung des Acetons wird die dabei erhaltene wäßrige Schicht mit 10 Litern Essigester extrahiert. Die Essigesterschicht wird zur Trockne eingedampft, was 8 g rohen Extrakt liefert.

Die getrockneten rohen Extrakte (20 g) aus dem Kulturfiltrat und dem Mycel werden vereinigt und durch wiederholte Säulenchromatographie über Kieselgel (Siebgröße 100-200) mit einem Eluiermittel, das aus Chloroform mit steigendem Anteil an Methanol, und zwar bis zu 10 % Methanol besteht, gereinigt. Nach dieser Methode erhält man 2 g bioaktive Fraktionen. Diese 2 g bioaktives Gemisch werden mit Petroläther (40-60° ) angerieben, was 1,5 g öligen inaktiven Rückstand und 360 mg bioaktiven Niederschlag liefert. Dieser Niederschlag wird in einem kleinen Volumen Methanol gelöst und durch eine ®Sephadex LH-20-Säule (70 x 3,5 cm) geschickt, wobei die Fraktionen jeweils mit Methanol eluiert werden. Dabei gewinnt man ungefähr 260 mg halbreines Antibiotikumgemisch, und dieses wird durch Dünnschichtchromatographie (0,5 mm dicke Kieselgelplatte) in einem aus 10 % Methanol in Chloroform bestehenden Lösungsmittelsystem weitergereinigt. Auf diese Weise erhält man 150 mg durch Chromatographie, Spektralanalyse und seine physikalisch-chemischen Eigenschaften identifiziertes Chalcomycin, 10 mg bioaktives Material und 12 mg Swalpamycin.

Das Swalpamycin wird durch chemische Analyse und spektroskopische Methoden identifiziert.

**Patentansprüche**

**Patentansprüche für folgende Vertragsstaaten : BE, CH, DE, FR, GB, GR, IT, LI, LU, NL, SE**

1. Eine Verbindung der Formel I

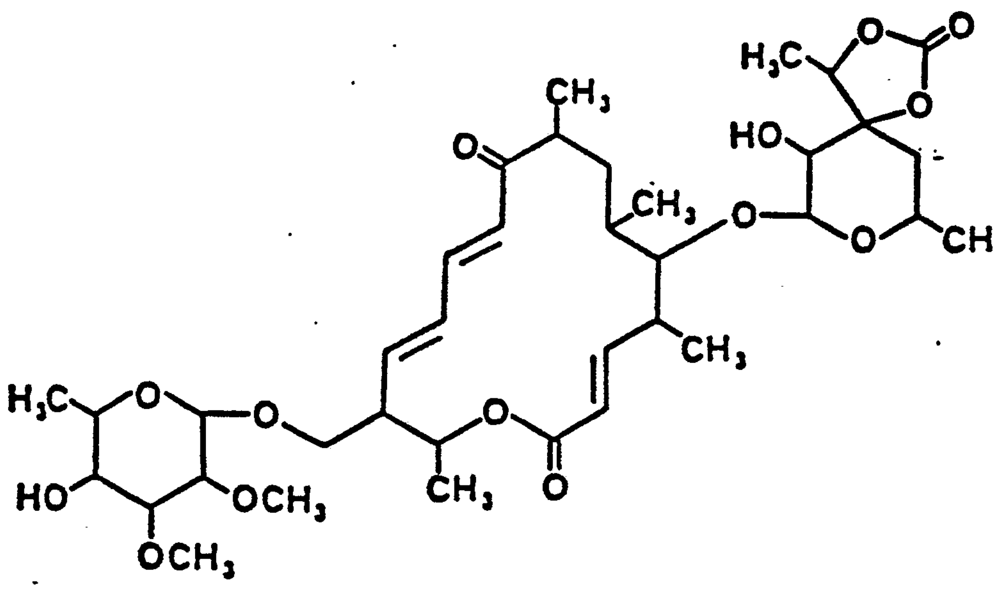

(I)

2. Verfahren zur Herstellung einer Verbindung der Formel I, dadurch gekennzeichnet, daß man den Mikroorganismus-Stamm Streptomyces species Y-84,30967 (DSM 3740) oder eine seiner Varianten oder Mutanten unter aeroben Bedingungen bei einem pH von 6 bis 7 und einer Temperatur von 20 bis 40 °C in einem üblichen, Kohlenstoff- und Stickstoffquellen, anorganische Nährsalze und ggfs. Spurenelemente enthaltenden Nährmedium fermentiert, und die erfindungsgemäße Verbindung durch Extraktion des Kulturfiltrats und des Mycels mit organischen Lösungsmitteln oder durch Anwendung von Adsorptionsmethoden auf das Kulturfiltrat und anschließende Anwendung von chromatographischen Methoden auf den Lösungsmittelextrakt bzw. das Eluat isoliert und reinigt.

3. Verfahren nach Anspruch 2, dadurch gekennzeichnet, daß man die Fermentation des Mikroorganismus

Streptomyces sp. Y-84,30967 (DSM 3740) als Submersfermentation bei einem pH von 6,5, einer Temperatur von 25-30 °C und einer Dauer von 40-45 Stunden durchführt.

**4.** Verfahren gemäß Anspruch 2, dadurch gekennzeichnet, daß man das Kulturfiltrat bei einem pH von 6,0 bis 7,5 mit einem mit Wasser nicht mischbaren organischen Lösungsmittel extrahiert, das Mycel mit Aceton extrahiert, den Extrakt zur Entfernung des Acetons eindampft, mit Wasser verdünnt und die wäßrige Phase mit einem mit Wasser nicht mischbaren organischen Lösungsmittel extrahiert und aus den vereinigten Lösungsmittelextrakten die erfindungsgemäße Verbindung durch Kieselgel-Säulenchromatographie und Dünnschichtchromatographie isoliert und reinigt.

**5.** Arzneimittel, gekennzeichnet durch einen Gehalt an der Verbindung gemäß Anspruch 1.

**6.** Verwendung einer Verbindung gemäß Anspruch 1 zur Herstellung von Arzneimitteln mit Wirkung gegen grampositive Bakterien, Mykoplasmen und Actinomyceten.

**7.** Streptomyces species Y-84,30967 (DSM 3740).

**Patentansprüche für folgende Vertragsstaaten : AT, ES**

**1.** Verfahren zur Herstellung einer Verbindung der Formel I

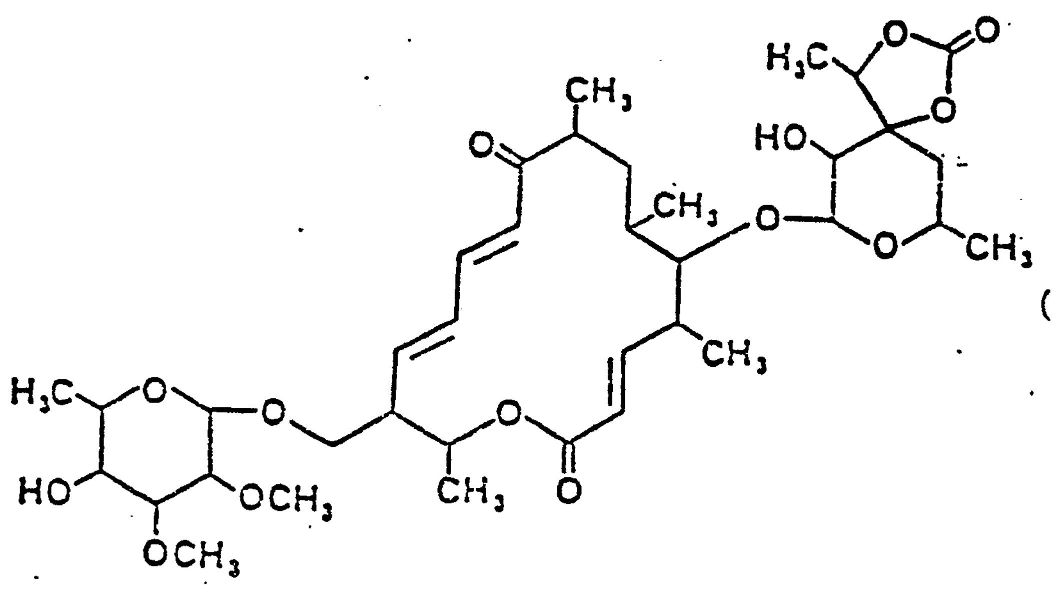

(I)

dadurch gekennzeichnet, daß man den Mikroorganismus-Stamm Streptomyces species Y-84,30967 (DSM 3740) oder eine seiner Varianten oder Mutanten unter aeroben Bedingungen bei einem pH von 6 bis 7 und einer Temperatur von 20 bis 40 °C in einem üblichen, Kohlenstoff- und Stickstoffquellen, anorganische Nährsalze und ggfs. Spurenelemente enthaltenden Nährmedium fermentiert, und die erfindungsgemäße Verbindung durch Extraktion des Kulturfiltrats und des Mycels mit organischen Lösungsmitteln oder durch Anwendung von Adsorptionsmethoden auf das Kulturfiltrat und anschließende Anwendung von chromatographischen Methoden auf den Lösungsmittelextrakt bzw. das Eluat isoliert und reinigt.

**2.** Verfahren nach Anspruch 1, dadurch gekennzeichnet, daß man die Fermentation des Mikroorganismus Streptomyces sp. Y-84,30967 (DSM 3740) als Submersfermentation bei einem pH von 6,5, einer Temperatur von 25-30 °C und einer Dauer von 40-45 Stunden durchführt.

**3.** Verfahren gemäß Anspruch 1, dadurch gekennzeichnet, daß man das Kulturfiltrat bei einem pH von 6,0 bis 7,5 mit einem mit Wasser nicht mischbaren organischen Lösungsmittel extrahiert, das Mycel mit Aceton extrahiert, den Extrakt zur Entfernung des Acetons eindampft, mit Wasser verdünnt und die wäßrige Phase mit einem mit Wasser nicht mischbaren organischen Lösungsmittel extrahiert und aus

den vereinigten Lösungsmittelextrakten die erfindungsgemäße Verbindung durch Kieselgel-Säulenchromatographie und Dünnschichtchromatographie isoliert und reinigt.

4. Verwendung einer Verbindung gemäß Anspruch 1 zur Herstellung von Arzneimitteln mit Wirkung gegen grampositive Bakterien, Mykoplasmen und Actinomyceten.

**Claims**
**Claims for the following Contracting States : BE, CH, DE, FR, GB, GR, IT, LI, LU, NL, SE**

1. A compound of the formula I

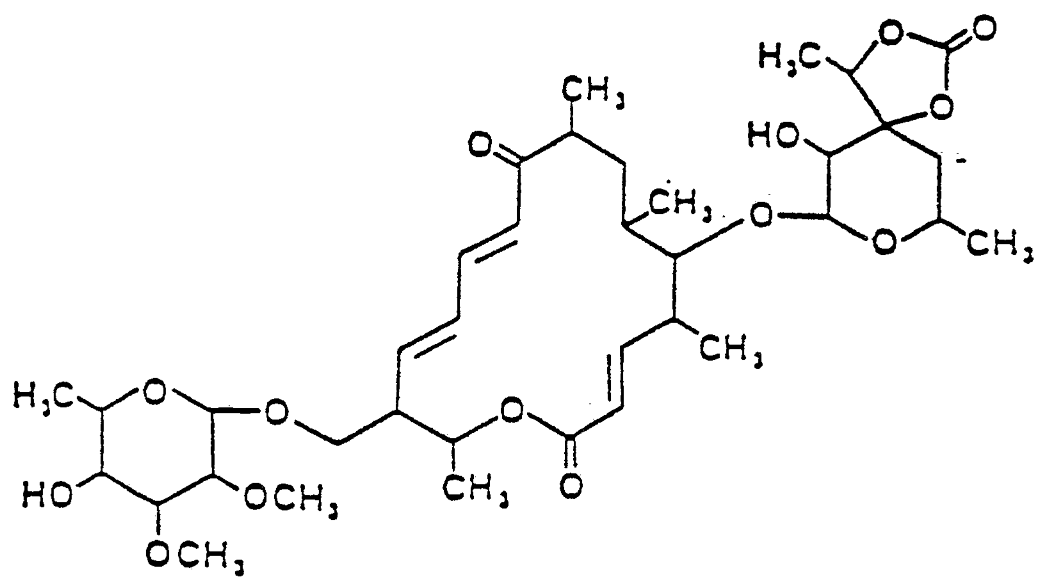

(I)

2. A process for the preparation of a compound of the formula I, which comprises fermentation of the microorganism strain Streptomyces species Y-84,30967 (DSM 3740), or one of its variants or mutants, under aerobic conditions, at a pH of 6 to 7 and a temperature of 20 to 40 °C, in a customary nutrient medium containing sources of carbon and nitrogen, inorganic nutrient salts and, where appropriate, trace elements, and isolation and purification of the compound according to the invention by extraction of the culture filtrate and of the mycelium with organic solvents, or by application of adsorption methods to the culture filtrate, and subsequent application of chromatographic methods to the solvent extract or the eluate.

3. The process as claimed in claim 2, wherein the fermentation of the microorganism Streptomyces sp. Y-84,30967 (DSM 3740) is carried out as submerged fermentation at a pH of 6.5 and a temperature of 25-30 °C, and with a duration of 40-45 hours.

4. The process as claimed in claim 2, wherein the culture filtrate is extracted at a pH of 6.0 to 7.5 with an organic solvent which is immiscible with water, the mycelium is extracted with acetone, the extract is evaporated to remove the acetone, and is diluted with water, and the aqueous phase is extracted with an organic solvent which is immiscible with water, and the compound according to the invention is isolated and purified from the combined solvent extracts by silica gel column chromatography and thin-layer chromatography.

5. A medicinal agent containing a compound as claimed in claim 1.

6. The use of a compound as claimed in claim 1 for the preparation of medicinal agents acting against Gram-positive bacteria, mycoplasmas and Actinomycetes.

7. Streptomyces species Y-84,30967 (DSM 3740).

**Claims for the following Contracting States : AT, ES**

1. A process for the preparation of a compound of the formula I

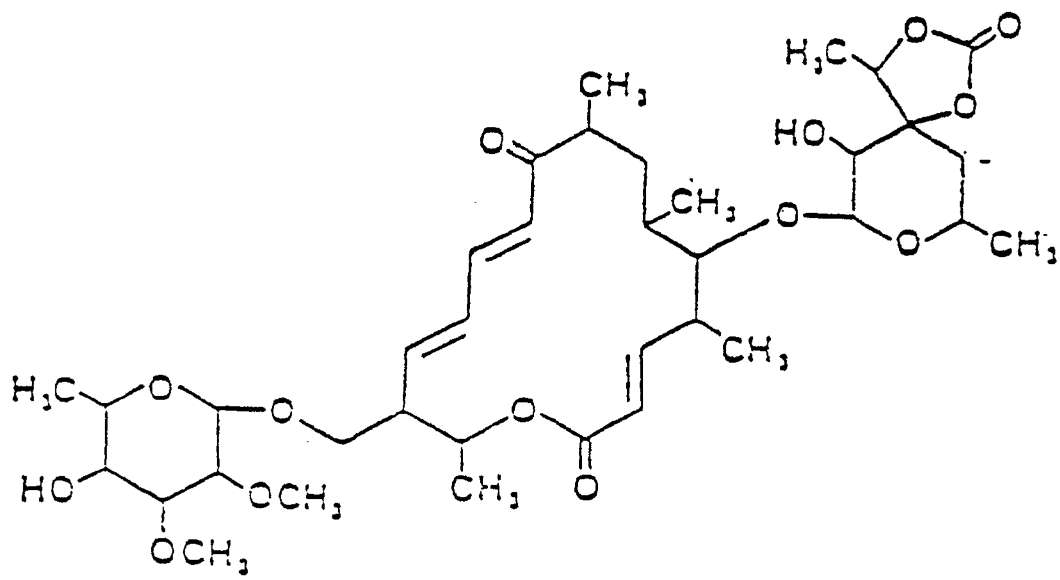

(I)

which comprises fermentation of the microorganism strain Streptomyces species Y-84,30967 (DSM 3740), or one of its variants or mutants, under aerobic conditions, at a pH of 6 to 7 and a temperature of 20 to 40°C, in a customary nutrient medium containing sources of carbon and nitrogen, inorganic nutrient salts and, where appropriate, trace elements, and isolation and purification of the compound according to the invention by extraction of the culture filtrate and of the mycelium with organic solvents, or by application of adsorption methods to the culture filtrate, and subsequent application of chromatographic methods to the solvent extract or the eluate.

2. The process as claimed in claim 1, wherein the fermentation of the microorganism Streptomyces sp. Y-84,30967 (DSM 3740) is carried out as submerged fermentation at a pH of 6.5 and a temperature of 25-30°C, and with a duration of 40-45 hours.

3. The process as claimed in claim 1, wherein the culture filtrate is extracted as a pH of 6.0 to 7.5 with an organic solvent which is immiscible with water, the mycelium is extracted with acetone, the extract is evaporated to remove the acetone, and is diluted with water, and the aqueous phase is extracted with an organic solvent which is immiscible with water, and the compound according to the invention is isolated and purified from the combined solvent extracts by silica gel column chromatography and thin-layer chromatography.

4. The use of a compound as claimed in claim 1 for the preparation of medicinal agents acting against Gram-positive bacteria, mycoplasmas and Actinomycetes.

**Revendications**

**Revendications pour les Etats contractants suivants : BE, CH, DE, FR, GB, GR, IT, LI, LU, NL, SE**

1. Composé de formule I

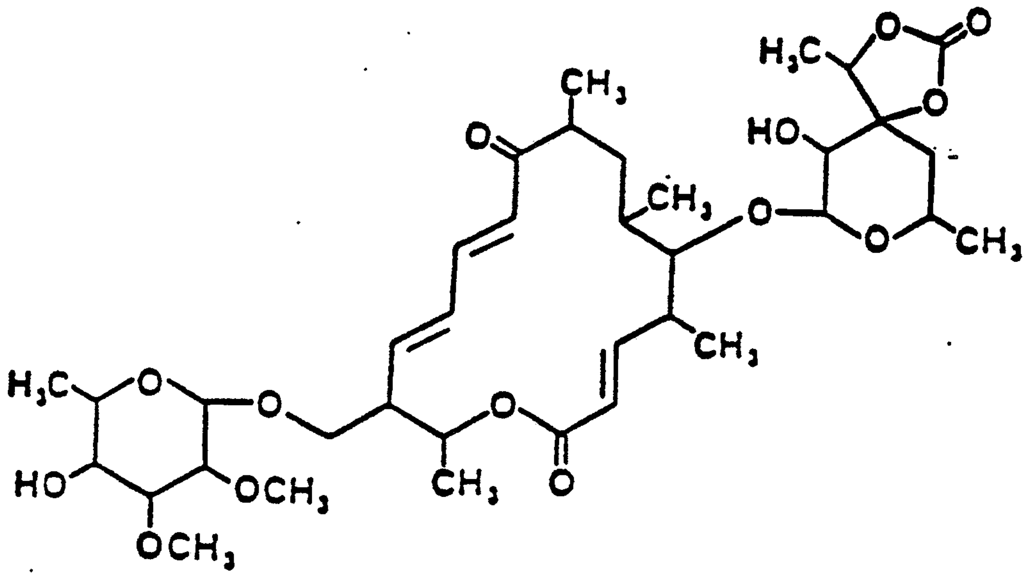

(I)

2. Procédé de préparation d'un composé de formule I, caractérisé en ce qu'on fait fermenter la souche de microorganisme Streptomyces Y-84,30967 (DSM 3740) ou une de ses variantes ou un de ses mutants, dans des conditions aérobies à pH 6-7 et à une température de 20 à 40 ° C, dans un milieu nutritif usuel renfermant des sources de carbone et d'azote, des sels nutritifs minéraux et éventuellement des oligoéléments, et en ce qu'on isole et purifie le composé de l'invention, par extraction du filtrat de culture et du mycélium avec des solvants organiques ou par mise en oeuvre de techniques d'adsorption sur le filtrat de culture, puis par application de procédés chromatographiques à l'extrait par solvant ou à l'éluat.

3. Procédé selon la revendication 2, caractérisé en ce qu'on réalise la fermentation du microorganisme Streptomyces Y-84,30967 (DSM 3740) sous forme d'une fermentation submergée, à pH 6,5, à une température de 25-30 ° C et pendant 40 à 45 heures.

4. Procédé selon la revendication 2, caractérisé en ce qu'on extrait le filtrat de culture, à pH 6,0-7,5, avec un solvant organique non miscible à l'eau, on extrait le mycélium avec de l'acétone, on concentre l'extrait pour éliminer l'acétone, on le dilue à l'eau et on extrait la phase aqueuse avec un solvant organique non miscible à l'eau, puis on isole, à partir des extraits réunis, et purifie le composé de l'invention, par chromatographie sur colonne de gel de silice et chromatographie sur couche mince.

5. Médicament, caractérisé en ce qu'il contient un composé selon la revendication 1.

6. Utilisation d'un composé selon la revendication 1 pour la fabrication de médicaments actifs contre les bactéries gram-positives, les mycoplasmes et les actinomycètes.

7. Streptomyces Y-84,30967 (DSM 3740).

**Revendications pour les Etats contractants suivants : AT, ES**

1. Procédé de préparation d'un composé de formule I

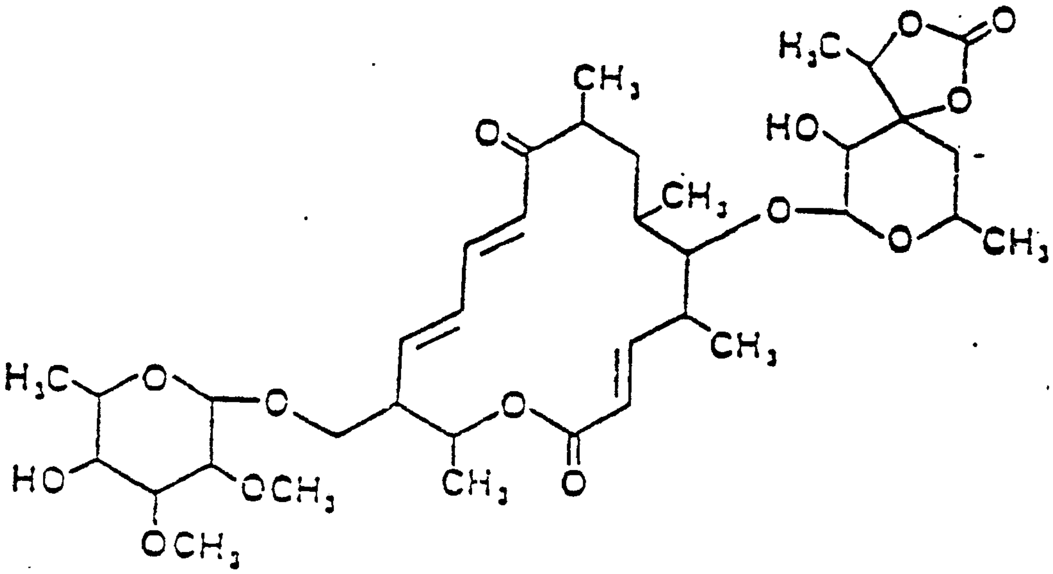

(I)

caractérisé en ce qu'on fait fermenter la souche de microorganisme Streptomyces Y-84,30967 (DSM 3740) ou une de ses variantes ou un de ses mutants, dans des conditions aérobies à pH 6-7 et à une température de 20 à 40 °C, dans un milieu nutritif usuel renfermant des sources de carbone et d'azote, des sels nutritifs minéraux et éventuellement des oligoéléments, et en ce qu'on isole et purifie le composé de l'invention, par extraction du filtrat de culture et du mycélium avec des solvants organiques ou par mise en oeuvre de techniques d'adsorption sur le filtrat de culture, puis par application de procédés chromatographiques à l'extrait par solvant ou à l'éluat.

2. Procédé selon la revendication 1, caractérisé en ce qu'on réalise la fermentation du microorganisme Streptomyces Y-84,30967 (DSM 3740) sous forme d'une fermentation submergée, à pH 6,5, à une température de 25-30 °C et pendant 40 à 45 heures.

3. Procédé selon la revendication 1, caractérisé en ce qu'on extrait le filtrat de culture, à pH 6,0-7,5, avec un solvant organique non miscible à l'eau, on extrait le mycélium avec de l'acétone, on concentre l'extrait pour éliminer l'acétone, on le dilue à l'eau et on extrait la phase aqueuse avec un solvant organique non miscible à l'eau, puis on isole, à partir des extraits réunis, et purifie le composé de l'invention, par chromatographie sur colonne de gel de silice et chromatographie sur couche mince.

4. Utilisation d'un composé selon la revendication 1 pour la fabrication de médicaments actifs contre les bactéries gram-positives, les mycoplasmes et les actinomycètes.

FIG.1

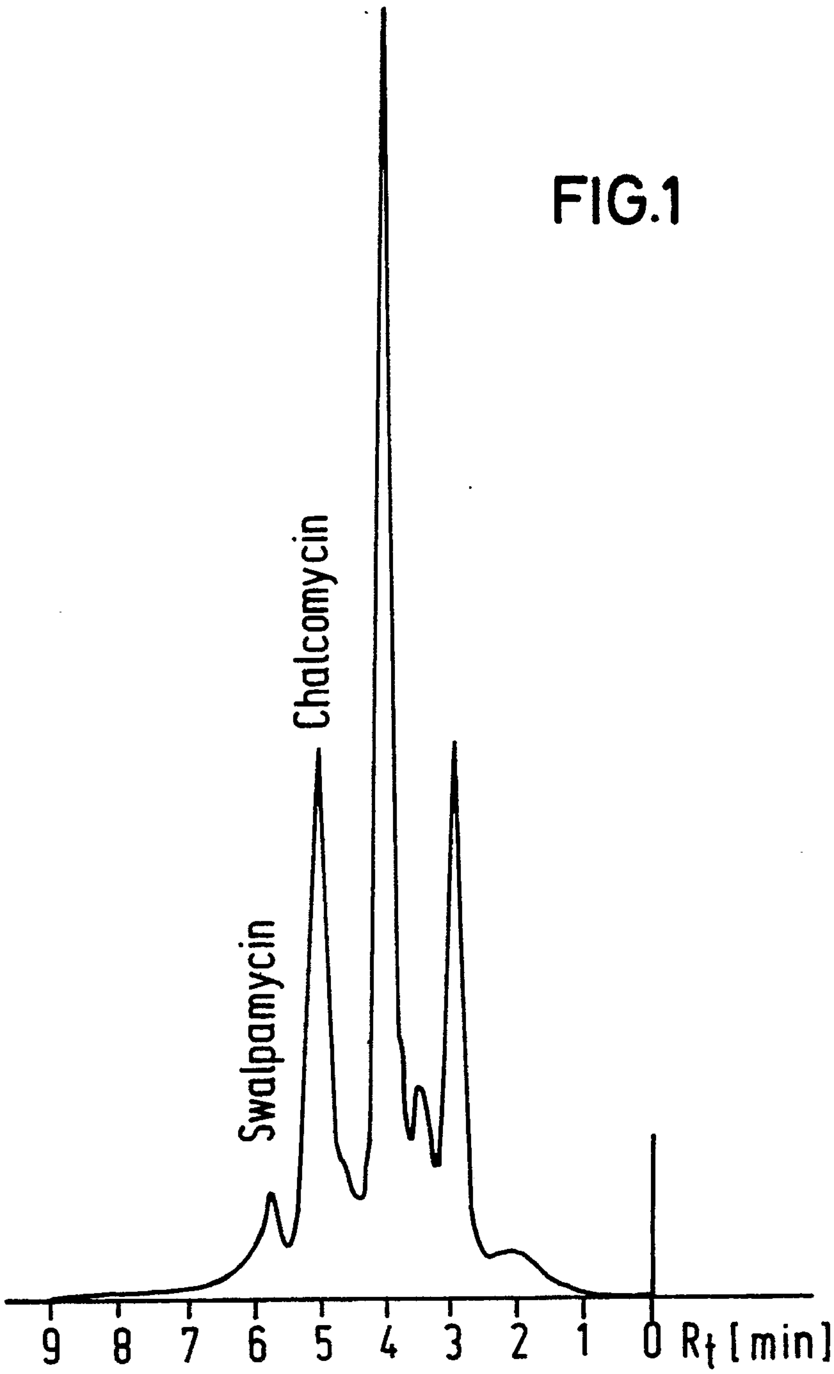

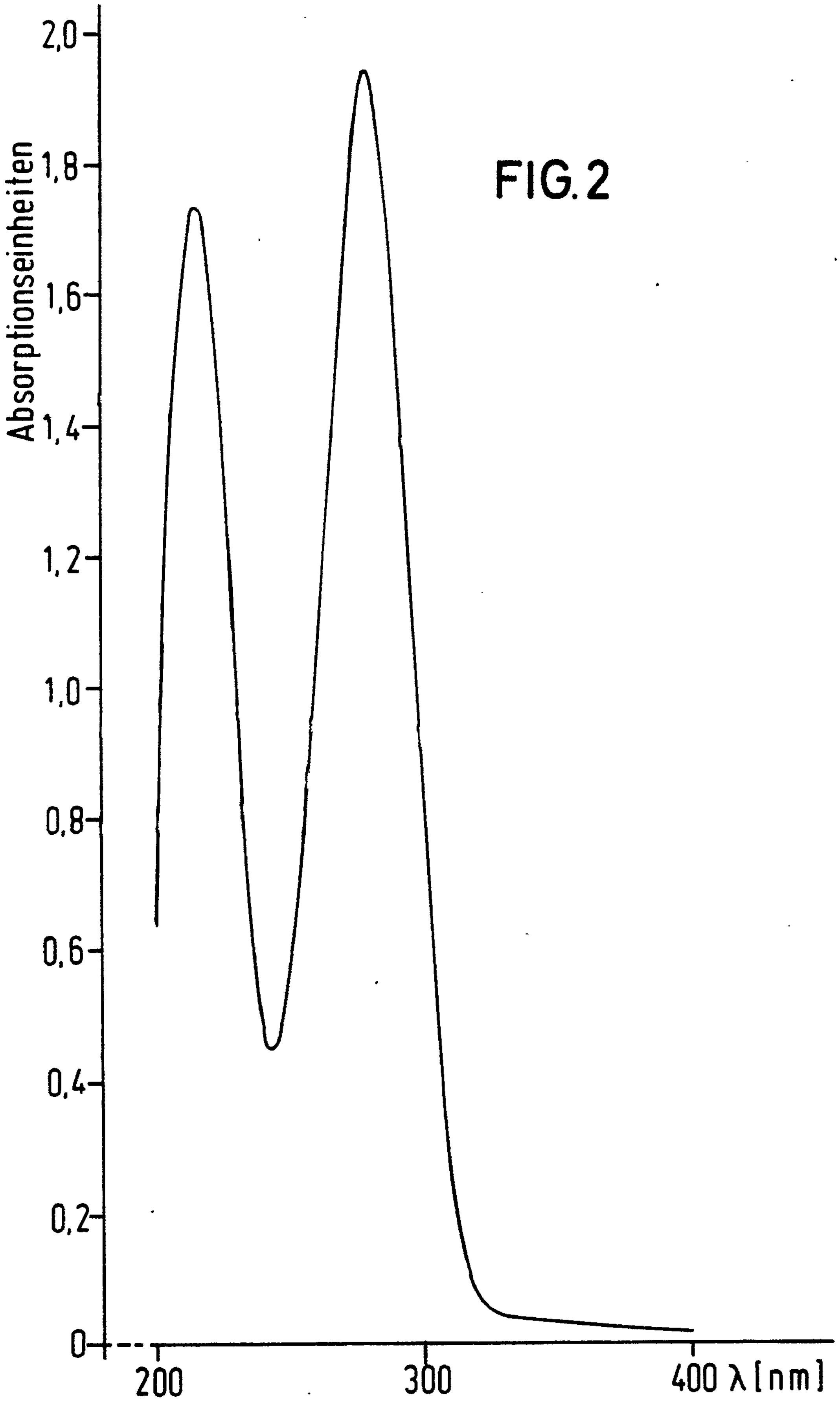
2,0
1,8
1,6
1,4
1,2
1,0
0,8
0,6
0,4
0,2
0
Absorptionseinheiten
200
300
400 λ [nm]

FIG. 2

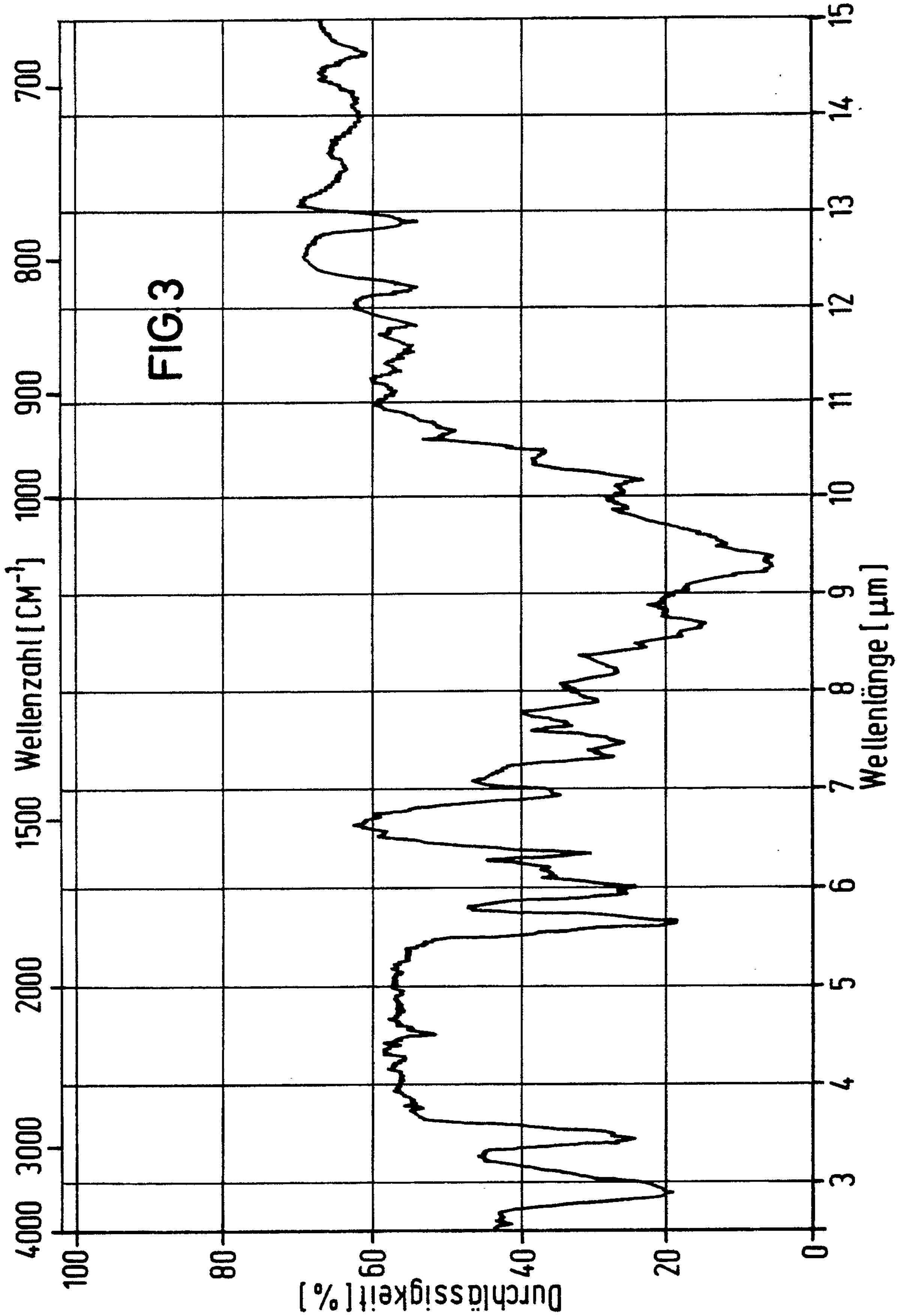
FIG.3
Wellenzahl [CM-1]
4000
3000
2000
1500
1000
900
800
700
Wellenlänge [µm]
3
4
5
6
7
8
9
10
11
12
13
14
15
Durchlässigkeit [%]
0
20
40
60
80
100

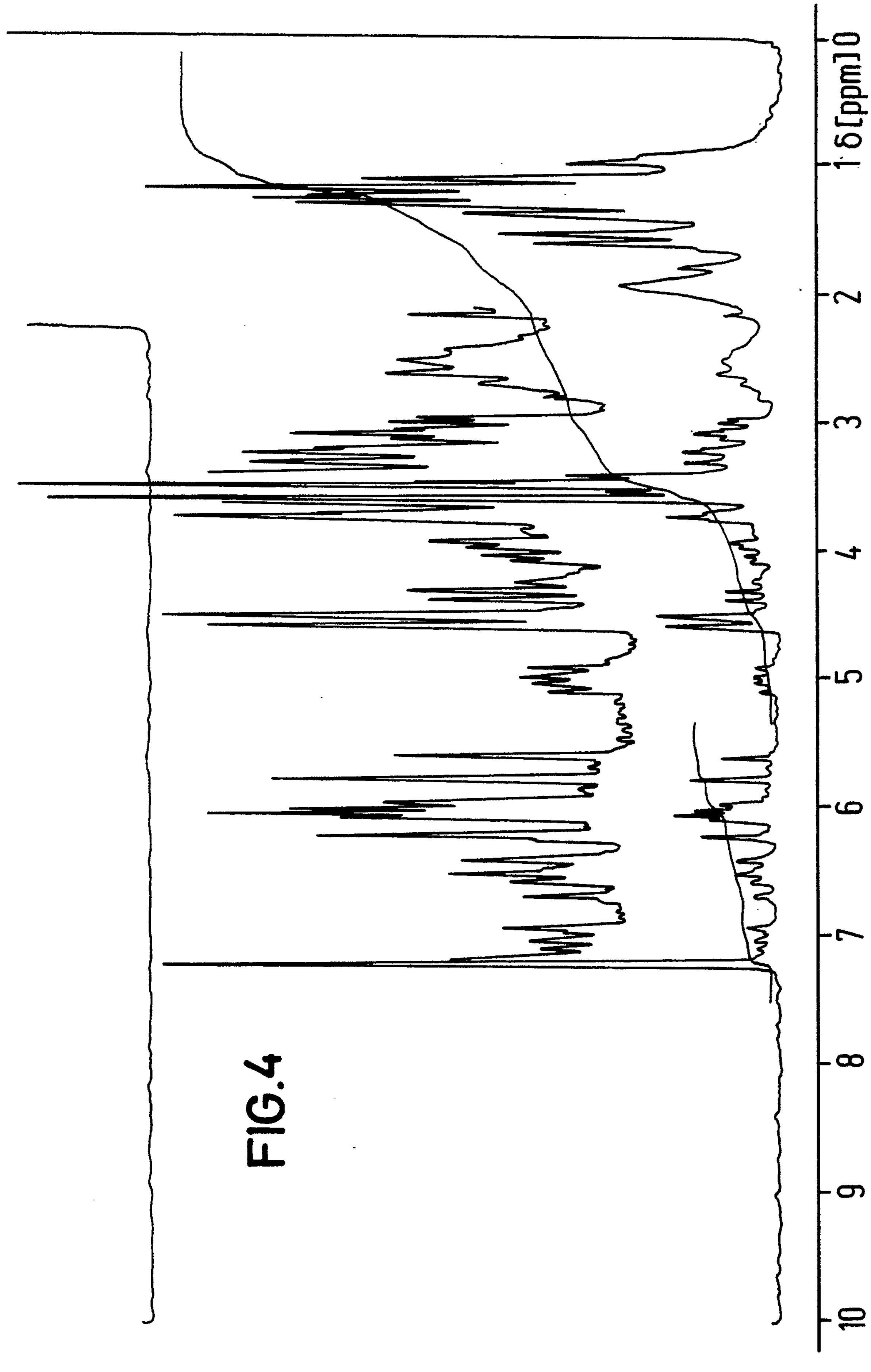


FIG.4

FIG. 5